(19) Europäisches Patentamt
European Patent Office
Office européen des brevets

(11) Veröffentlichungsnummer: **0 046 554**
**B1**

(12) **EUROPÄISCHE PATENTSCHRIFT**

(45) Veröffentlichungstag der Patentschrift: **14.12.83**

(51) Int. Cl.³: **C 07 C 109/14**, C 07 C 149/14, C 07 C 149/32, A 61 K 31/15

(21) Anmeldenummer: **81106354.4**

(22) Anmeldetag: **17.08.81**

(54) **N-substituierte Brenztraubensäurehydrazone, Verfahren zu ihrer Herstellung und Arzneimittel, die diese Verbindungen enthalten.**

(30) Priorität: **23.08.80 DE 3031842**

(43) Veröffentlichungstag der Anmeldung: **03.03.82 Patentblatt 82/9**

(45) Bekanntmachung des Hinweises auf die Patenterteilung: **14.12.83 Patentblatt 83/50**

(84) Benannte Vertragsstaaten: **AT BE CH DE FR GB IT LI LU NL SE**

(56) Entgegenhaltungen:
**EP - A - 0 001 144**
**DE - A - 2 643 303**
**DE - A - 2 726 210**

**Chemical Abstracts Band 92, Nr. 9, 3. März 1980 Columbus, Ohio, USA R. HAECKEL et al "Hydrazonopropionic acids, a new class of non-hormonal hypoglycemic compounds" Seite 36, Spalte 1, Abstract Nr. 69438r**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Heerdt, Ruth, Dr. rer. nat., Wallstädter Strasse 62, D-6800 Mannheim-Feudenheim (DE)**
Erfinder: **Wolff, Hans-Peter, Dr. rer. nat., Rebenweg 24, D-6945 Hirschberg-Grosssachsen (DE)**
Erfinder: **Kaiser, Fritz, Dr. rer. nat., Hans-Holbein-Strasse 10, D-6840 Lampertheim (DE)**
Erfinder: **Schaumann, Wolfgang, Prof. Dr. med., Mönchhofstrasse 58, D-6900 Heidelberg (DE)**
Erfinder: **Kühnle, Hans, Dr. rer. nat., Karlsruherstrasse 17, D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 046 554 B1

N-substituierte Brenztraubensäurehydrazone, Verfahren zu ihrer Herstellung und Arzneimittel, die diese Verbindungen enthalten

In den deutschen Patentanmeldungen DE-OS Nrn. 2643303 und 2739380 sind N-substituierte Brenztraubensäurehydrazone der Formel I beschrieben. Einige Verbindungen aus diesen Anmeldungen wurden von R. Haeckel und M. Oellerich („Biochem. Soc. Trans.", 7 [1979], 749) eingehend auf ihre pharmakologische Wirksamkeit untersucht. Die Verbindungen der Formel I zeigen eine ausgeprägte hypoglykämische Wirkung. Der Rest X bedeutet dabei in allen Fällen einen Valenzstrich oder eine niedere Alkylengruppe. Es wurde nun überraschend gefunden, dass die Einführung eines Heteroatoms in den Rest X zu Verbindungen führt, welche die Absorption von Glucose aus dem Intestinaltrakt in einem Dosisbereich hemmen, in dem die blutzuckersenkende Wirkung noch nicht oder nur unwesentlich auftritt. Die Verbindungen eignen sich daher zur Behandlung von Krankheiten, bei denen nach Aufnahme von kohlenhydrathaltigen Nahrungsmitteln starke und langanhaltende Hyperglykämien auftreten. Insbesondere sind sie geeignet als Therapeutika für die Indikationen Diabetes, Prädiabetes, Adipositas und Atherosklerose.

Gegenstand der vorliegenden Erfindung sind daher neue Brenztraubensäurehydrazone der allgemeinen Formel I

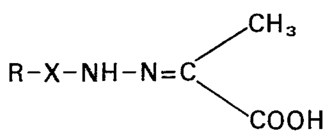

(I)

in der

R ein Wasserstoffatom, einen gegebenenfalls niederalkylsubstituierten Arylrest oder einen geradkettigen oder verzweigten, gesättigten oder ungesättigten Alkylrest, der durch einen niederen Alkoxy-Cycloalkyl- oder einen gegebenenfalls niederalkylsubstituierten Arylrest substituiert sein kann, und

X die Gruppe $-A-B-$ bedeutet, mit der Massgabe, dass A an das Stickstoffatom und B an den Rest R gebunden sind,

wobei

A eine geradkettige oder verzweigte Alkylengruppe mit 2 bis 8 Kohlenstoffatomen darstellt, mit einem geradkettigen Teilstück von mindestens 2 Kohlenstoffatomen, das B mit dem Stickstoffatom verbindet, und

B ein Sauerstoff- oder ein Schwefelatom bedeuten,

deren physiologisch unbedenklichen Salze, Ester und Amide, Verfahren zur Herstellung derselben, sowie ihre Verwendung zur Herstellung von Arzneimitteln mit antidiabetischer Wirkung.

Unter einem Arylrest sind aromatische Kohlenwasserstoffe mit 6 bis 14 Kohlenstoffatomen, insbesondere der Phenylrest, zu verstehen.

Unter der Alkylgruppe des Substituenten R sollen geradkettige oder verzweigte, gesättigte oder ungesättigte Kohlenwasserstoffreste mit 1 bis 5 Kohlenstoffatomen verstanden werden. In diesem Sinne bevorzugt sind der Methyl-, der Äthyl-, der Propyl-, der Allyl- und der Methallylrest.

Der Begriff Cycloalkylgruppe umfasst Carbocyclen mit 5 bis 7 Kohlenstoffatomen. Vorzugsweise ist die Cyclohexylgruppe gemeint.

Unter substituierten Arylresten werden solche aromatische Kohlenwasserstoffe mit 6 bis 14 Kohlenstoffatomen verstanden, die in einer oder mehreren der möglichen Positionen Halogen, niedere Alkyl- oder niedere Alkoxygruppen tragen. Dabei versteht man unter Halogen vorzugsweise Fluor, Chlor und Brom.

Unter niederen Alkylgruppen sind in allen Fällen geradkettige oder verzweigte Reste mit 1 bis 4 Kohlenstoffatomen zu verstehen. Diese Definition gilt auch für den Alkylrest der niederen Alkoxygruppen. Vorzugsweise finden als niederer Alkylrest die Methylgruppe und als niederer Alkoxyrest die Methoxy- und die Äthoxygruppe Verwendung.

Unter geradkettigen und verzweigten Alkylengruppen des Restes A seien insbesondere die folgenden verstanden:

$-(CH_2)_x-$, mit x = 2 bis 6, und 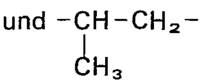

Bevorzugt sind solche Verbindungen, in denen B ein Sauerstoffatom bedeutet.

Ferner sind Gegenstand der Erfindung sämtliche stereoisomeren Formen einer Verbindung der allgemeinen Formel I, die aufgrund der bei manchen Verbindungen vorhandenen asymmetrischen Kohlenstoffatome oder Doppelverbindungen (C–C, C–N) auftreten können.

Die Verbindungen der allgemeinen Formel I können nach an sich bekannten Verfahren hergestellt werden, vorzugsweise indem man ein Hydrazin der allgemeinen Formel II

$$R-X-NH-NH_2 \qquad (II)$$

in der R und X die obige Bedeutung haben, mit einem Propionsäure-Derivat der allgemeinen Formel III

$$CH_3-C(Y,Y')-COR' \qquad (III),$$

in der Y und Y' Halogen- oder Alkoxygruppen oder zusammen ein Sauerstoffatom darstellen und R' Hydroxy, eine niedere Alkoxy- oder eine gegebenenfalls substituierte Aminogruppe bedeuten,

umsetzt und anschliessend gegebenenfalls die Säure in ihr Salz, einen Ester oder ein Amid überführt bzw. die Säure aus ihren Derivaten herstellt.

Halogen der Reste Y und Y' der Formel III bedeutet Fluor, Chlor, Brom und Jod, vorzugsweise jedoch Chlor und Brom. Alkoxygruppen der Reste Y, Y' und R' sind Gruppen mit 1 bis 4 Kohlenstoffatomen, bevorzugt ist die Methoxy- und Äthoxygruppe.

Bei diesem Verfahren wird das substituierte Hydrazin II oder ein entsprechendes Salz in einem geeigneten polaren Lösungsmittel (z.B. Wasser, einem niederen Alkohol oder Essigsäure) mit einem Propionsäurederivat III oder vorzugsweise mit dessen Salzen versetzt und gegebenenfalls mit Hilfe eines Puffers, z.B. Natriumacetat, auf ein schwach saures pH gebracht. Die Reaktion läuft bei Raumtemperatur ab, kann jedoch auch unter Erwärmen durchgeführt werden. Die Hydrazone I können als schwerlösliche Verbindungen aus dem Reaktionsmedium abfiltriert oder mit geeigneten Lösungsmitteln extrahiert werden, z.B. unpolaren Lösungsmitteln.

Gegebenenfalls kann auch in einem Eintopfverfahren das substituierte Hydrazin II z.B. aus einem Amin mit Hydroxylamin-O-sulfonsäure hergestellt und nach Zugabe des Propionsäurederivates III das gewünschte Hydrazon ausgefällt werden.

Die substituierten Hydrazine bzw. ihre Salze sind zum Teil neue Verbindungen. Eine Reindarstellung ist im allgemeinen nicht erforderlich, so dass die anfallenden Rohprodukte eingesetzt werden können. Die Hydrazine können nach an sich bekannten Verfahren hergestellt werden, beispielsweise durch Umsetzung von Hydrazin mit entsprechenden Alkylhalogeniden.

Als physiologisch unbedenkliche Salze kommen insbesondere Alkali-, Erdalkali- oder Ammoniumsalze (sowie gegebenenfalls Salze mit blutzuckersenkenden Biguaniden) in Frage. Die Herstellung dieser Salze erfolgt in an sich bekannter Weise, beispielsweise durch Umsetzung mit den entsprechenden freien Basen, Carbonaten oder Alkoholaten.

Die bei dem oben aufgeführten Verfahren als Zwischenprodukte auftretenden Ester können isoliert oder gegebenenfalls direkt zu den entsprechenden Carbonsäuren verseift werden. Umgekehrt können die erhaltenen Carbonsäuren wieder nach an sich bekannten Methoden zu den gewünschten Estern umgesetzt werden. Die Verseifungen der Ester werden vorzugsweise in alkalischem Medium durchgeführt.

Als Ester der Carbonsäuren der allgemeinen Formel I sind im Sinne der Erfindung prinzipiell die Reaktionsprodukte der Carbonsäuren mit alkoholen zu verstehen. Bevorzugt sind jedoch die niederen einwertigen Alkohole wie Methanol, Äthanol, Propanol oder Isopropanol.

Die erfindungsgemässen Amide der allgemeinen Formel I können nach an sich bekannten Methoden aus den Carbonsäuren oder ihren reaktiven Derivaten durch Umsetzung mit Aminen hergestellt werden. Als Aminkomponenten kommen z.B. Ammoniak, Mono- und Dialkylamine sowie Aminosäuren in Frage, wobei p-Aminobenzoesäure, Anthranilsäure, Phenylalanin, α- und β-Alanin, Serin, Valin, Glycin, Arginin und viele mehr zu nennen sind.

Als erfindungsgemässe antidiabetische Zubereitungen kommen alle üblichen oralen und parenteralen Applikationsformen in Frage, beispielsweise Tabletten, Kapseln, Dragées, Sirupe, Lösungen, Suspensionen, Tropfen, Suppositorien. Zu diesem Zweck vermischt man den Wirkstoff mit festen oder flüssigen Trägerstoffen und bringt sie anschliessend in die gewünschte Form. Feste Trägerstoffe sind z.B. Stärke, Lactose, Methylzellulose, Talkum, hochdisperse Kieselsäure, höher-molekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykole). Für die orale Applikation geeignete Zubereitungen können gewünschtenfalls Geschmacks- und Süssstoffe enthalten.

Als Injektionsmedium kommt vorzugsweise Wasser zur Anwendung, welches die bei Injektionslösungen üblichen Zusätze wie Stabilisierungsmittel, Lösungsvermittler und/oder Puffer enthält. Derartige Zusätze sind z.B. Acetat- oder Tartratpuffer, Äthanol, Komplexibildner (wie Äthylendiamintetraessigsäure und deren nichttoxische Salze) oder hochmolekulare Polymere (wie flüssiges Polyäthylenoxid) zur Viskositätsregulierung.

Zur Bekämpfung von Krankheiten, bei denen nach Aufnahme von kohlendydrathaltigen Nahrungsmiteln starke und langanhaltende Hyperglykämien auftreten, werden die pharmakologisch aktiven Verbindungen der allgemeinen Formel I in Einzeldosen von 1 bis 600, vorzugsweise von 50 bis 500 mg, angewandt, wobei diese Einzeldosen je nach Bedarf ein- oder mehrmals pro Tag verabreicht werden können.

Im Sinne der vorliegenden Anmeldung ist ausser den in den Beispielsen genannten Verbindungen und allen übrigen Kombinationen der einzelnen Definitionen der Substituenten R, X, A und B noch die folgende Säure bevorzugt:

2-[2-(β-Methylcinnamyloxy)äthylhydrazono]propionsäure.

Pharmakologisch weniger wirksame Verbindungen der allgemeinen Formel I sind als wertvolles Zwischenprodukt für die Herstellung von hochwirksamen Verbindungen anzusehen.

Anhand der nachfolgenden Beispiele wird die Erfindung näher erläutert, wobei dies keine Einschränkung des Erfindungsgegenstandes darstellen soll:

*Beispiel 1*

*2-(2-p-Tolyloxyäthylhydrazono)propionsäure*

11,8 g (58 mmol) 2-p-Tolyloxyäthylhydrazinhydrochlorid werden in 100 ml Wasser gelöst und unter Rühren bei Raumtemperatur mit einer Lösung von 5,1 g (58 mmol) Brenztraubensäure und 7,5 g Natriumacetat in 35 ml Wasser versetzt. Dabei scheidet sich ein Öl ab, das nach Abdekantieren der wässerigen Reaktionsphase durch Anreiben mit Wasser zur Kristallisation gebracht wird. Die Substanz wird abgesaugt, unter Zugabe von 30 ml 2N-Natronlauge in Wasser gelöst und die Lösung langsam mit 2N-Salzsäure versetzt. Der ausgefallene Niederschlag wird abgesaugt und getrocknet. Ausbeute: 8,9 g (65% d. Th.); Fp. 57-59° C.

In analoger Weise erhält man durch Umsetzung von Brenztraubensäure

a) mit 2-Hydroxyäthylhydrazinsulfat
2-(2-Hydroxyäthylhydrazono)propionsäure
Fp. 83-85° C

b) mit 2-(2-Äthoxyäthoxy)äthylhydrazinsulfat
2-[2-(2-Äthoxyäthoxy)äthylhydrazono]-propionsäure
Fp. 51-55° C

c) mit 2-Phenoxyäthylhydrazinhydrochlorid
2-(2-Phenoxyäthylhydrazono)propionsäure
Fp. 90-92° C (Isopropanol/Wasser) (Hydrat)

d) mit 3-Phenoxypropylhydrazinhydrochlorid
2-(3-Phenoxypropylhydrazono)propionsäure
Fp. 56-58° C (Isooctan/Toluol) (Hydrat)

*Beispiel 2*

*Natrium-2-(2-phenoxypropylhydrazono)-propionat*

14,6 g (72 mmol) 2-Phenoxypropylhydrazinhydrochlorid werden in 100 ml Wasser gelöst und mit einer Lösung von 6,7 g (76 mmol) Brenztraubensäure und 14 g Natriumacetat in 30 ml Wasser gemischt. Es bildet sich ein Öl. Man rührt etwa 1 h, extrahiert das Öl mit Methylenchlorid, wäscht diese Lösung mit Wasser, trocknet sie mit Natriumsulfat und destilliert das Methylenchlorid im Vakuum ab. Den öligen Rückstand löst man in 50 ml Äthanol und gibt unter Rühren 13,4 ml einer 30%ig Natriummethylatlösung zu. Das Natrium-2-(2-phenoxypropylhydrazono)propionat scheidet sich ab, wird abgesaugt und zuerst mit wenig Äthanol, dann mit Äther gewaschen.
Ausbeute: 11,3 g (61% d. Th.); Fp. 209-211° C.

In analoger Weise erhält man durch Umsetzung von Brenztraubensäure

a) mit 2-(2-Methoxyäthoxy)äthylhydrazinsulfat und anschliessende Darstellung des Natriumsalzes
Natrium-2-[2-(-methoxyäthoxy)äthylhydrazono]propionat
Fp. 175-177° C

b) mit 2-Benzyloxyäthylhydrazinhydrochlorid und anschliessende Darstellung des Natriumsalzes
Natrium-2-(2-benzyloxyäthylhydrazono)-propionat
Fp. 175° C (Semihydrat)

c) mit 2-Allylthioäthylhydrazinhydrochlorid und anschliessende Darstellung des Natriumsalzes
Natrium-2-(2-allylthioäthylhydrazono)-propionat
Fp. 197° C (Zers.)

d) mit 2-Phenylthioäthylhydrazinhydrochlorid und anschliessende Darstellung des Natiumsalzes
Natrium-2-(2-phenylthioähylhydrazono)-propionat
Fp. 199-200° C

*Beispiel 3*

*2-(2-Allyloxyäthylhydrazono)propionsäure*

4,0 g (34 mmol) 2-Allyloxyäthylhydrazin werden in 20 ml Wasser gelöst und mit 3,1 g (36 mmol) Brenztraubensäure versetzt. Man lässt etwa 30 min stehen und extrahiert dann das Reaktionsgemisch mit Äther. Die Ätherextrakte werden mit einer Natriumacetat/Salzsäure-Pufferlösung (pH 3,5) gewaschen, über Natriumsulfat getrocknet und bei Raumtemperatur im Vakuum eingedampft. Man erhält als Rückstand ein farbloses Öl.
Ausbeute: 3,2 g (50% d. Th.).
NMR-Spektrum ($d_6$DMSO)

δ: 1,79 ppm ($C\underline{H_3}-\overset{\|}{C}-COOH$)
7,33 ppm ($N\underline{H}$)

In analoger Weise erhält man durch Umsetzung von Brenztraubensäure

a) mit 6-Methoxyhexylhydrazin
2-(6-Methoxyhexylhydrazono)propionsäure
farbloses Öl
NMR-Spektrum ($d_6$DMSO)

δ: 1,78 ppm ($C\underline{H_3}-\overset{\|}{C}-COOH$)
7,33 ppm ($N\underline{H}$)

b) mit 5-Äthoxypenthylhydrazin
2-(5-Äthoxypentylhydrazono)propionsäure farbloses Öl
NMR-Spektrum ($d_6$DMSO)

δ: 1,80 ppm ($C\underline{H_3}-\overset{\|}{C}-COOH$)
7,28 ppm ($N\underline{H}$)

c) mit 4-Propoxybutylhydrazin
2-(4-Propoxybutylhydrazono)propionsäure
farbloses Öl
NMR-Spektrum ($d_6$DMSO)

δ: 1,78 ppm ($C\underline{H_3}-\overset{\|}{C}-COOH$)
7,32 ppm ($N\underline{H}$)

d) mit 2-(2-Cyclohexyläthoxy)äthylhydrazin
2-[2-(2-Cyclohexyläthoxy)äthylhydrazono]propionsäure
farbloses Öl
NMR-Spektrum ($d_6$DMSO)

δ: 1,80 ppm ($C\underline{H_3}-\overset{\|}{C}-COOH$)
7,33 ppm ($N\underline{H}$)

e) mit 3-Benzyloxypropylhydrazin
2-(3-Benzyloxypropylhydrazono)propionsäure
farbloses Öl
NMR-Spektrum ($d_6$DMSO)

δ: 1,76 ppm ($C\underline{H_3}-\overset{\|}{C}-COOH$)
7,28 ppm ($N\underline{H}$)

f) mit 2-(2-Methoxyäthylthio)äthylhydrazin
2-[2-(2-Methoxyäthylthio)äthylhydrazono]propionsäure
farbloses Öl
NMR-Spektrum ($d_6$DMSO)

δ: 1,80 ppm ($C\underline{H_3}-\overset{\|}{C}-COOH$)
7,47 ppm ($N\underline{H}$)

*Versuchsprotokoll*

Zum Nachweis der pharmakologischen Eigenschaften der erfindungsgemässen Verbindungen wurden die blutzuckersenkende Wirkung und die Resorptionshemmung in getrennten Versuchen bestimmt und mit zwei strukturell verwandten Verbindungen verglichen.

1. Testverfahren zur Ermittlung der Schwellendosis

Als experimentelles Modell zur Ermittlung der Schwellendosis der Blutglukose senkenden Wirkung der zu untersuchenden Substanzen wurde das nüchterne Meerschweinchen herangezogen. Den für die Untersuchungen verwendeten Individuen wurde 16 h vor Versuchsbeginn die Nahrung entzogen. Die Tiere blieben während des gesamten Versuchszeitraums ohne Futter, hatten jedoch freien Zugang zum Trinkwasser.

Die Substanzgabe erfolgte durch i.p.-Applikation einer Lösung des K-Salzes bei pH 7,4.

Eine paralell dazu mitgeführte Kontrollgruppe erhielt eine 0,9%ige NaCl-Lösung verabreicht.

Die Blutabnahmen zur Bestimmung der Glukosekonzentration wurden unmittelbar vor der Substanzapplikation sowie in einstündigen Abständen bis zur 4. Stunde nach der Substanzgabe vorgenommen. Dazu wurde eine Ohrvene mit einer Kanüle Nr. 18 vorsichtig punktiert und der austretende Blutstropfen wurde mit einer 10 µl Kapillare entnommen.

Die Bestimmung der Blutglukosekonzentration erfolgte mittels der spezifischen Hexokinasemethode im Hämolysat. Dazu wurde die 10 µl-Blutprobe in eine Stabilisatorlösung einpippetiert, die Digitonin als Hämolysebeschleuniger und Maleinimid als Glykolysehemmer enthielt. Anschliessend wurde aus dem so gewonnenen Hämolysat ein Aliquot entnommen und nach dem Prinzip der Hexokinasemethode am LKB 8600 (Fa. LKB, Bromma, Schweden) bestimmt.

Als Schwellendosis wurde diejenige Dosis bezeichnet, die bei der vorgegebenen Anzahl der Versuchstiere pro Dosisgruppe (N=4) gerade eine signifikante Senkung der Nüchternglykämie bewirkt ($p < 0{,}05$).

2. Testverfahren zur Ermittlung der resorptionshemmenden Wirkung

Zur Prüfung der Wirkung auf die Glukoseresorption wurde eine orale Glukosebelastung durchgeführt. Dazu wurde einem aus 10 Tieren bestehenden Kollektiv von nüchternen Meerschweinchen die zu prüfende Substanz wie beschrieben als K-Salz i.p. verabreicht. Gleichzeitig erhielten die Tiere 1 g Glukose als 20%ige Lösungsmittel einer Schlundsonde oral appliziert.

Nach Abnahme eines Vorwertes wurde den Tieren in einem engmaschigen Raster (20 min) bis 200 min Blut zur Bestimmung der Glukosekonzentration wie beschrieben entnommen.

Eine parallel dazu mitgeführte Kontrollgruppe erhielt ebenfalls 1 g Glukose *p.o.* sowie eine entsprechende Menge 0,9%iger NaCl-Lösung i.p. verabreicht.

Die Bestimmung der Blutglukosekonzentration erfolgte wie oben beschrieben mittels der Hexokinasemethode im Hämolysat.

Als Messgrösse für die Glukoseresorption wurde die mit Hilfe der Trapezsummenformel berechnete Fläche unter der Konzentrationszeitkurve herangezogen. Die in % ausgedrückte Differenz der Fläche der Kontroll- und Substanzgruppe wurde als Mass für die Hemmung der Glukoseresorption angegeben.

Als Referenzsubstanzen wurden folgende Verbindungen getestet:

A) 2-(Phenäthylhydrazono)propionsäure (Beispiel 1 aus US-PS Nr. 4136196)

B) 2-(2-Cyclohexyläthylhydrazono)propionsäure (Beispiel 1 aus US-PS Nr. 4206231)

Die Ergebnisse sind in der folgenden Tabelle zusammengestellt:

*Tabelle*

| Beispielnummer | Schwellendosis Meerschweinchen (mg/kg) i.p. | Resorptionshemmung Dosis (mg/kg) i.p. | Resorptionshemmung Hemmung (%) |
|---|---|---|---|
| A | 10-15 | 10 | 0 |
| B | 15 | 15 | 0 |
| 1 | 25 | 40 | 25 |
| 1b | 25 | 40 | 98 |
| 2a | >50 | 40 | 37,03 |
| 2b | >50 | 40 | 49,86 |
| 2c | 10-25 | 10 | 16 |
| 2d | 25-50 | 40 | 33 |
| 3 | 25 | 20 | 9 |
| 3a | 20 | 20 | 11 |
| 3b | 20-25 | 20 | 34 |
| 3d | >50 | 40 | 15 |
| 3e | >50 | 40 | 44 |
| 3f | 25 | 20 | 17,3 |

## Patentansprüche

1. Brenztraubensäurehydrazone der allgemeinen Formel (I)

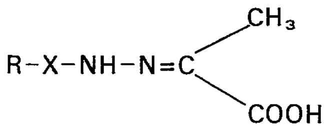

(I)

in der

R ein Wasserstoffatom, einen gegebenenfalls niederalkylsubstituierten Arylrest oder einen geradkettigen oder verzweigten, gesättigten oder ungesättigten Alkylrest, der durch einen niederen Alkoxy-, Cycloalkyl- oder einen gegebenenfalls niederalkylsubstituierten Arylrest substituiert sein kann, und

X die Gruppe $-A-B-$ bedeutet, mit der Massgabe, dass A an das Stickstoffatom und B an den Rest R gebunden sind,

wobei

A eine geradkettige oder verzweigte Alkylengruppe mit 2 bis 8 Kohlenstoffatomen darstellt, mit einem geradkettigen Teilstück von mindestens 2 Kohlenstoffatomen, das B mit dem Stickstoffatom verbindet, und

B ein Sauerstoff- oder ein Schwefelatom bedeuten,

sowie deren physiologisch unbedenkliche Salze, Ester und Amide.

2. Verbindungen der allgemeinen Formel (I) gemäss Anspruch 1, in denen R die angegebenen Bedeutungen hat und X eine der Gruppen $-O(CH_2)_{2\ \text{bis}\ 6}-$, $-OCH(CH_3)CH_2-$ oder $-SCH_2CH_2$ bedeutet, beziehungsweise deren physiologisch unbedenklichen Salze, Ester und Amide.

3. Verbindungen der allgemeinen Formel (I) gemäss einem der Ansprüche 1 oder 2, in denen X die angegebenen Bedeutungen hat und R Phenyl, Benzyl, Äthyl oder Methoxyäthyl bedeutet, beziehungsweise deren physiologish unbedenklichen Salze, Ester und Amide.

4. 2-(2-Phenoxyäthylhydrazono)propionsäure, beziehungsweise deren physiologisch unbedenklichen Salze, Ester und Amide.

5. 2-[2-(2-Methoxyäthoxy)äthylhydrazono]propionsäure, beziehungsweise deren physiologisch unbedenklichen Salze, Ester und Amide.

6. 2-(2-Benzyloxyäthylhydrazono)propionsäure, beziehungsweise deren physiologisch unbedenklichen Salze, Ester und Amide.

7. 2-(5-Äthoxypentylhydrazono)propionsäure, beziehungsweise deren physiologisch unbedenklichen Salze, Ester und Amide.

8. Verfahren zur Herstellung von Verbindungen der allgemeinen Formel (I):

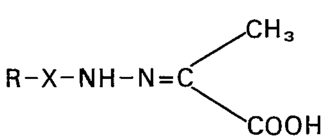

(I)

in der

R ein Wasserstoffatom, einen gegebenenfalls niederalkylsubstituierten Arylrest oder einen geradkettigen oder verzweigten, gesättigten oder ungesättigten Alkylrest, der durch einen niederen Alkoxy-, Cycloalkyl- oder einen gegebenenfalls niederalkylsubstituierten Arylrest substituiert sein kann, und

X die Gruppe $-A-B-$ beudetet, mit der Massgabe, dass A an das Stickstoffatom und B an den Rest R gebunden sind,

wobei

A eine geradkettige oder verzweigte Alkylengruppe mit 2 bis 8 Kohlenstoffatomen darstellt, mit einem geradkettigen Teilstück von mindestens 2 Kohlenstoffatomen, das B mit dem Stickstoffatom verbindet, und

B ein Sauerstoff- oder ein Schwefelatom bedeuten,

sowie deren physiologisch unbedenklichen Salzen, Estern und Amiden,

dadurch gekennzeichnet, dass man in an sich bekannter Weise ein Hydrazin der allgemeinen Formel (II):

$$R-X-NH-NH_2 \qquad (II)$$

in der R und X die obige Bedeutung haben, mit einem Propionsäurederivat der allgemeinen Formel (III):

$$CH_3-C(Y,Y')-COR' \qquad (III)$$

in der Y und Y' Halogen- oder Alkoxygruppen oder zusammen ein Sauerstoffatom darstellen und R' Hydroxy, eine niedere Alkoxy- oder eine gegebenenfalls substituierte Aminogruppe bedeuten,

umsetzt und anschliessend gegebenenfalls die Säure in ihr Salz, einen Ester oder ein Amid überführt bzw. die Säure aus ihren Derivaten herstellt.

9. Brenztraubensäurehydrazone der allgemeinen Formel (I) oder deren physiologisch unbedenklichen Salze, Ester und Amide zur Bekämpfung von Diabetes, Prädiabetes, Adipositas oder Atheroskleros.

10. Arzneimittel enthaltend Verbindungen der allgemeinen Formel (I), beziehungsweise deren physiologisch unbedenklichen Salze, Ester oder Amide sowie an sich bekannte pharmakologisch verträgliche Trägerstoffe.

## Revendications

1. Hydrazones de l'acide pyruvique de formule générale (I):

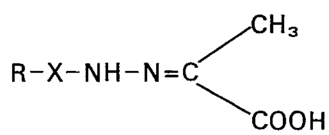

(I)

dans laquelle

R est un atome d'hydrogène, un reste aryle éventuellement substitué par de l'alkyle inférieur ou un reste alkyle saturé ou insaturé à chaîne droite ou ramifiée, pouvant être substitué par un reste alcoxy inféfieur, cycloalkyle ou aryle, ce dernier

éventuellement substitué par de l'alkyle inférieur, et

X est le groupe $-A-B-$, sous réserve que A soit lié à l'atome d'azote et B au reste R,

et où

A est un groupe alkylène à chaîne droite ou ramifiée ayant de 2 à 8 atomes de carbone, renfermant un tronçon à chaîne droite d'au moins 2 atomes de carbone, reliant B à l'atome d'azote, et

B est un atome d'oxygène ou de soufre,

ainsi que leurs sels, esters et amides physiologiquement acceptables.

2. Composés de formule générale (I) selon la revendication 1, dans lesquels R a les significations indiquées et X est un des groupes $-O-(CH_2)_{2-6}-$, $-OCH(CH_2)CH_2-$ ou $-SCH_2CH_2-$, ainsi que les sels, esters et amides physiologiquement acceptables de ces composés.

3. Composés de formule générale (I) selon l'une des revendications 1 ou 2, dans lesquels X a les significations indiquées et R représente le phényle, le benzyle, l'éthyle ou le méthoxyéthyle, ainsi que les sels, esters et amides physiologiquement acceptables de ces composés

4. L'acide 2-(2-phénoxyéthylhydrazono)propionique et ses sels, esters et amides physiologiquement acceptables.

5. L'acide 2-[2-(2-méthoxyéthoxy)éthylhydrazono]proponique et ses sels, esters et amides physiologiquement acceptables.

6. L'acide 2-(2-benzyloxyéthylhydrazono)-propionique et ses sels, esters et amides physiologiquement acceptables.

7. L'acide 2-(5-éthoxypentylhydrazono)propionique et ses sels, esters et amides physiologiquement acceptables.

8. Procédé pour la préparation de composés de formule générale (I):

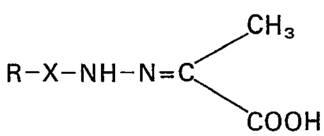

(I)

dans laquelle

R est un atome d'hydrogène, un reste aryle éventuellement substitué par de l'alkyle inférieur ou un reste alkyle saturé ou insaturé à chaîne droite ou ramifiée, pouvant être substitué par un reste alcoxy inférieur, cycloalkyle ou aryle, ce dernier éventuellement substitué par de l'alkyle inférieur, et

X est le groupe $-A-B-$, sous réserve que A soit lié à l'atome d'azote et B au reste R,

et où

A est un groupe alkylène à chaîne droite ou ramifiée ayant de 2 à 8 atomes de carbone, renfermant un tronçon à chaîne droite d'au moins 2 atomes de carbone, reliant B à l'atome d'azote, et

B est un atome d'oxygène ou de soufre,

ainsi que leurs sels, esters et amides physiologiquement acceptables,

caractérisé en ce que, de façon connue en soi, on fait réagir une hydrazine de formule générale (II):

$$R-X-NH-NH_2 \qquad (II)$$

dans laquelle

R et X ont la signification indiquée ci-dessus, avec un dérivé d'acide propionique de formule générale (III):

$$CH_3-C(Y,Y')-COR' \qquad (III)$$

dans laquelle

Y et Y' sont des groupes halogène ou alcoxy ou représentent ensemble un atome d'oxygène, et

R' est de l'hydroxyle, un groupe alcoxy inférieur ou un groupe amino éventuellement substitué,

et ensuite on transforme éventuellement l'acide en son sel, un ester ou un amide ou bien on prépare l'acide à partir de ses dérivés.

9. Hydrazones de l'acide pyruvique de formule générale (I) ou les sels, esters ou amides physiologiquement acceptables pour le traitement du diabète, du prédiabète, de l'adiposité ou de l'athérosclérose.

10. Médicament contenant des composés de formule générale (I), ou leurs sels, esters ou amides physiologiquement acceptables, ainsi que des substances de support connues pharmacologiquement acceptables.

## Claims

1. Pyruvic acid hydrazones of the general formula (I):

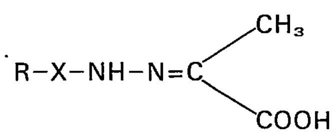

(I)

in which

R signifies a hydrogen atom, an optionally lower alkyl-substituted aryl radical or a straight-chained or branched, saturated or unsaturated alkyl radical, which can be substituted by a lower alkoxy, cycloalkyl or an optionally lower alkyl-substituted aryl radical, and

X signifies the group $-A-B-$, with the proviso that A is attached to the nitrogen atom and B to the radical R,

whereby

A signifies a straight-chained or branched alkylene group with 2 to 8 carbon atoms, with a straight-chaines part of at least 2 carbon atoms which joins B with the nitrogen atom,

and B signifies an oxygen or a sulphur atom,

as well as their physiologically acceptable salts, esters and amides.

2. Compounds of general formula (I) according to claim 1, in which R has the given meanings and X signifies the groups $-O(CH_2)_{2-6}$, $-OCH-(CH_3)CH_2-$ or $-SCH_2CH_2-$ or their physiologically acceptable salts, esters and amides.

3. Compounds of general formula (I) according to one of claims 1 or 2, in which X has the given meanings and R signifies phenyl, benzyl, ethyl or methoxyethyl or their physiologically acceptable salts, esters and amides.

4. 2-(2-Phenoxyethylhydrazono)propionic acid or its physiologically acceptable salts, esters and amides.

5. 2-[2-(2-Methoxyethoxy)ethylhydrazono]-propionic acid or its physiologically acceptable salts, esters and amides.

6. 2-(2-Benzyloxyethylhydrazono)propionic acid or its physiologically acceptable salts, esters and amides.

7. 2-(5-Ethoxypentylhydrazono)propionic acid or its physiologically acceptable salts, esters and amides.

8. Process for the preparation of compounds of the general formula (I):

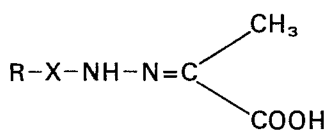

(I)

in which

R signifies a hydrogen atom, an optionally lower alkyl-substituted aryl radical or a straight-chained or branched, saturated or unsaturated alkyl radical, which can be substituted by a lower alkoxy, cycloalkyl or an optionally lower alkyl-substituted aryl radical, and

X signifies the group −A−B−, with the proviso that A is joined to the nitrogen atom and B to the radical R,

whereby

A represents a straight-chained or branched alkylene group with 2 to 8 carbon atoms, with a straight-chained part of at least 2 carbon atoms which joins B with the nitrogen atoms, and B signifies an oxygen or sulphur atom,

as well as of their physiologically acceptable salts, esters and amides,

charaterised in that, in per se known manner, one reacts a hydrazine of the general formula (II):

$$R-X-NH-NH_2 \qquad (II)$$

in which

R and X have the above-given meaning, with a propionic acid derivative of the general formula (III):

$$CH_3\text{-}C(Y,Y')-COR' \qquad (III)$$

in which

Y and Y' represent halogen or alkyl groups or together an oxygen atoms, and

R' signifies hydroxyl, a lower alkoxy or a possibly substituted amino group,

and subsequently optionnally converts the acid into its salt, an ester or an amide or prepares the acid from its derivatives.

9. Pyruvic acid hydrazones of general formula (I) or their physiologically acceptable salts, esters and amides for the combating of diabetes, prediabetes, adipositas or atherosclerosis.

10. Medicaments containing compounds of general formula (I) or their physiologically acceptable salts, esters or amides, as well as per se known pharmacologically acceptable carrier materials.